# EUROPEAN PATENT APPLICATION

(11) **EP 2 848 285 A1**
(43) Date of publication of application: **18.03.2015**
(21) Application number: 13184452.4
(22) Date of filing: 13.09.2013
(51) Int. Cl.: A61Q 7/00, A61K 8/98

(54) **Compositions comprising medium supernatant of a stem cell culture**

(71) Applicant: Blue Horizon International LLC, New York, NY 10014 (US)
(72) Inventor: The designation of the inventor has not yet been filed
(74) Representative: Vossius & Partner

(57) **Abstract**

The present invention relates to the use of an aqueous cosmetic composition comprising the medium supernatant of a stem cell culture for caring hair or skin. The present invention also relates to an aqueous pharmaceutical composition comprising the medium supernatant of a stem cell culture for use in the prevention or treatment of alopecia.

## Description

The present invention relates to the use of an aqueous cosmetic composition comprising the medium supernatant of a stem cell culture for caring hair or skin. The present invention also relates to an aqueous pharmaceutical composition comprising the medium supernatant of a stem cell culture for use in the prevention or treatment of alopecia.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Human hair grows everywhere on the body except for the soles of the feet and the palms of the hands, the lips, and the eyelids, apart from eyelashes. Like skin, hair is a stratified squamous, keratinized epithelium made of multi-layered, flat cells with overlying keratin (a protein), whose rope-like filaments provide structure and strength to the hair shaft.

Hair follows a specific growth cycle with three distinct and concurrent phases: anagen, catagen, and telogen phases. Each strand of hair on the human body is at its own stage of development. Once the cycle is complete, it restarts and a new strand of hair begins to form. The anagen phase is known as the growth phase. It begins in the papilla and can last from two to six years (A. Y. Elzouki, H. A. Harfi, H. Nazer, William Oh, F. B. Stapleton, R. J. Whitley (ed.). Textbook of clinical pediatrics (2ed.). Berlin: Springer. p.1489, and Braun-Falco, Otto (2000). Dermatology (2ed.). Berlin: Springer. p.1101). The span at which the hair remains in this stage of growth is determined by genetics. The longer the hair stays in the anagen phase, the faster and longer it will grow. During this phase, the cells in the papilla divide to produce new hair fibers (i.e. keratinous fibers of hair), and the hair follicle buries itself into the dermal layer of the skin to nourish the strand. About 85% of the hairs on one's head are in the anagen phase at any given time. Signals sent out by the body determine when the anagen phase ends and the catagen phase begins. The catagen phase, also known as the transitional phase, allows the hair follicle to, in a sense, renew itself. During this time, which lasts about two weeks, the hair follicle shrinks due to disintegration and the papilla detaches and "rests," cutting the hair strand off from its nourishing blood supply. Ultimately, the hair follicle is 1/6 its original length, causing the hair shaft to be pushed upward. While hair is not growing during this phase, the length of the terminal fibers increase when the follicle pushes them upward. During the telogen, or resting, phase the hair and follicle remain dormant anywhere from 1-4 months. Ten to fifteen percent of the hairs on one's head are in this phase of growth at any given time. The anagen phase begins again once the telogen phase is complete. The preceding hair strand is pushed up and out by the new, growing strand. The process causes the normal hair loss known as shedding. The head of hair is thus constantly replaced and, of the approximately 150,000 hairs which a head of hair comprises, approximately 10% are at rest and will be replaced in the months to come.

All mammals have some hair on their skin, even marine mammals which appear to be hairless. In mammals, the skin is the largest organ of the integumentary system made up of multiple layers of ectodermal tissue, and guards the underlying muscles, bones, ligaments and internal organs. Mammalian skin is composed of two primary layers: (i) the epidermis, which provides waterproofing and serves as a barrier to infection; and (ii) the dermis, which serves as a location for the appendages of skin. The dermis contains the hair follicles. Skin and its appendages ensure a number of critical functions necessary for animal survival. Skin protects animals from water loss, temperature change, radiation, trauma, and infections, and it allows animals to perceive their environment through tactile sense. Through camouflage, the skin provides protection against predators, and it also serves as decoration for social and reproductive behavior.

Adult skin is composed of a diverse organized array of cells emanating from different embryonic origins. In mammals, shortly after gastrulation, the neurectoderm cells that remain at the embryo surface become the epidermis, which begins as a single layer of unspecified progenitor cells. During development, this layer of cells forms a stratified epidermis (sometimes called interfollicular epidermis), the hair follicles (HRs), sebaceous glands, and, in nonhaired skin, the apocrine (sweat) glands. Mesoderm-derived cells contribute to the collagen-secreting fibroblasts of the underlying dermis, the dermovasculature that supplies nutrients to skin, arrector pili muscles that attach to each hair follicle (HF), the subcutaneous fat cells, and the immune cells that infiltrate and reside in the skin. Neural crest-derived cells contribute to melanocytes, sensory nerve endings of the skin, and the dermis of the head. Overall, approximately 20 different cell types reside within the skin.

In the adult, many different types of stem cells (SCs) function to replenish these various cell types in skin as it undergoes normal homeostasis or wound repair. Some SCs (e.g., those that replenish lymphocytes) reside elsewhere in the body. Others (e.g., melanoblasts and epidermal SCs) reside within the skin itself. Epidermal SCs possess two essential features common to all SCs: They are able to self-renew for extended periods of time, and they differentiate into multiple lineages derived from their tissue origin (see Blanpain and Fuchs (2006), Annu Rev Cell Dev Biol. 2006; 22: 339-373).

The adult skin epithelium is composed of molecular building blocks, each of which consists of a pilosebaceous unit (hair follicle and sebaceous gland) and its surrounding interfollicular epidermis. The interfollicular epidermis contains its own progenitor cells to ensure tissue renewal in the absence of injury, and hair follicle contain multipotent SCs that are activated at the start of a new hair cycle and upon wounding to provide cells for hair follicle regeneration and repair of the epidermis.

Natural hair loss (i.e. shedding) can be estimated, on average, at a few hundred individual hairs per day for a normal physiological state. This process of constant physical replacement undergoes a natural change. For example, with aging, the hair becomes finer and its cycles shorter. Hair has great social significance for human beings. Healthy hair indicates health and youth. Bald patches or lots of thinning of hair indicate an abnormal hair loss. There are many causes of abnormal hair loss. Women may notice hair loss after giving birth. People under a lot of stress can see noticeable hair loss. Furthermore, some diseases and medical treatments can cause hair loss. The most common cause of hair loss is a medical condition called hereditary hair loss. About 80 million men and women in the United States have this type of hair loss.

Hair loss, in particular alopecia, is in most cases due to disturbances in the above-described hair replacement cycle. These disturbances result first in the acceleration in the frequency of the cycles, at the expense of the quality of the hair and then of their quantity. The bulbs gradually become smaller and, at the same time, the latter become isolated by gradual thickening of the perifollicular collagen matrix and of the external connective tissue sheath. Revascularization about the hair follicle is therefore rendered more difficult cycle after cycle. Individual hairs regress, becoming smaller until no more than an unpigmented down, and this phenomenon results in a gradual thinning of the head of hair. Areas are preferentially affected, in particular the temporal or frontal regions in men and, in women, diffuse alopecia of the vertex is observed. The term "alopecia" also covers a whole family of conditions of the hair follicle having, as final consequence, the definitive loss, partial or general, of the hair. It relates more particularly to androgenic alopecia. In a large number of cases, early hair loss occurs in genetically predisposed individuals. This is then androchronogenetic alopecia; this form of alopecia concerns men in particular. Furthermore, it is known that certain factors, such as a hormone imbalance, a physiological stress or malnutrition, can accentuate the phenomenon. In addition, hair loss or detrimental change can be related to seasonal phenomena. Generally, any factor which influences these processes, namely the acceleration in the frequency of the cycles, the gradual reduction in size of the bulbs, the gradual thickening of the perifollicular collagen matrix, the thickening of the external connective tissue sheath and the reduction in vascularization, will have an effect on the growth of the hair follicles.

In the art the following drugs are used to treat and/or prevent abnormal hair loss: Minoxidil is applied to the scalp. It can stop hairs from getting thinner and stimulate hair growth on the top of the scalp. The U.S. Food and Drug Administration (FDA) has approved minoxidil to treat hair loss. It is currently the only hair re-growth product approved for men and women. A dermatologist may combine minoxidil with another treatment. Laser devices such as brushes, combs, and other hand-held devices that emit laser light, might stimulate hair growth. These devices might make hair look more youthful in some people. However, the long-term effectiveness and safety for these devices are not known. The FDA furthermore approved finasteride to treat men with hair loss. It comes in pill form and helps slow hair loss in most (about 88%) men. It helps stimulate hair re-growth in many (about 66%) men. Finasteride works by stopping the body from making a male hormone, dihydrotestosterone (DHT). If hair loss is caused by inflammation, corticosteroids may be may injected into the scalp. This can help stop the inflammation that happens when a person has alopecia areata. In addition surgical methods, such as hair transplantation, scalp reduction, scalp expansion or scalp flaps may be used to treat hair loss.

Despite these known means and methods there is an ongoing need to provide further means and methods for the prevention and treatment of hair loss. As discussed above, hair loss may be prevented or treated for cosmetic reasons (to improve the appearance of hair and skin) as well as for medical reasons (in order to prevent or treat a disease characterized by hair loss). Both aspects are addressed by the present invention.

Accordingly the present invention relates in a first aspect to the use of an aqueous cosmetic composition comprising the medium supernatant of a stem cell culture for caring hair or skin.

The first aspect of the present invention also relates to the use of the medium supernatant of a stem cell culture in an aqueous cosmetic composition for caring hair or skin.

It has to be understood that the medium supernatant of a stem cell culture is used in an amount which effective for caring hair or skin. The supernatant of a stem cell culture may either be directly used for the preparation of a cosmetic composition or further processed before use, such as fractioned or purified.

Caring hair or skin involves bringing the hair or skin to be cared into contact with the cosmetic composition. The preferred region of the skin is the scalp and the preferred hair is scalp hair. Consequently, the invention also pertains to an aqueous cosmetic composition which comprises the medium supernatant of a stem cell culture.

Cosmetic compositions are commonly defined by their intended use, as articles or compositions intended to be rubbed, poured, sprinkled, or sprayed on, introduced into (such as injected), or otherwise applied to the human body for cleansing, beautifying, promoting attractiveness, or altering the appearance. A corresponding definition can, for example, be found in the US Food, Drug, and Cosmetic Act, FD&C Act.

The term "cosmetic composition" therefore delimits the composition according to the invention from "pharmaceutical compositions" (which are described herein below). The terms "cosmetic composition" and "pharmaceutical composition" are mutually exclusive. In other words, a cosmetic composition according to the invention is for use in non-therapeutic applications while the pharmaceutical composition according to the invention is for use in therapeutic applications.

The cosmetic composition of the invention is an aqueous cosmetic composition. An aqueous cosmetic composition is a cosmetic composition in which one, preferably the main solvent is water. The aqueous phase thus comprises water and may optionally comprise an ingredient miscible in any proportion with water, such as lower C₁ to C₈ alcohols, for example ethanol or isopropanol, polyols, such as propylene glycol, glycerol or sorbitol, or else acetone or ether.

The particular formulation of the cosmetic composition according to the invention is not limited. Envisaged formulations include solutions, emulsions, creams, milks, gels such as hydrogels, ointments, suspensions, dispersions, foams, sprays and shampoos. For this purpose, the cosmetic composition according to the invention may further comprise cosmetically acceptable diluents and/or carriers. Choosing appropriate carriers and diluents in dependency of the desired formulation is within the skills of the skilled person. Suitable cosmetically acceptable diluents and carriers are well known in the art and include agents referred to in Bushell et al. (WO 2006/053613).

Moreover, the cosmetic composition can contain any cosmetically acceptable ingredients. For example, the cosmetic composition of the invention may comprise, without limitation, one or more further ingredients such as keratolytic compounds (e.g. salicylic acid, urea), moisturizers (e.g. artificial or natural oils), anti-irritants (e.g. allantoin, (-)-α-bisabolol, anti-oxidants (e.g. tocopherol), chelating agents (e.g. EDTA), preservative agents (e.g. hydroxybenzoic acid and derivatives thereof), sun protection agents (e.g. benzophenone-3), pigments (e.g. titanium dioxide) and fragrances.

A preferred cosmetic composition is formulated for topical application to the skin (preferably the scalp) and hair (preferably scalp hair). For this topical application the composition can have preferably the form of an aqueous, or aqueous/alcoholic solution or suspension; an oily suspension or solution; an emulsion (water-in-oil or oil-in-water) or dispersion with a liquid or semi-liquid consistency obtained by dispersion of a fatty phase in an aqueous phase (O/W) or vice versa (W/O); a dispersion or emulsion with a soft consistency; oran aqueous or aqueous/alcoholic gel.. The composition can also have the form of microcapsules or microparticles, or of vesicular dispersions of ionic and/or nonionic type. It is also possible to envisage a composition in the form of a foam or alternatively in the form of an aerosol composition also comprising a pressurized propellant.

In particular, the composition for application to the scalp or scalp hair can be provided in the form of a hair care lotion, for example for daily or twice-weekly application, of a shampoo or of a hair conditioner, in particular for twice-weekly or weekly application, of a liquid or solid soap for cleaning the scalp for daily application, of a product for shaping the hairstyle (lacquer, hairsetting product, styling gel), of a treatment mask, of a cream or of a foaming gel for cleaning the hair. It can also be provided in the form of a hair dye or mascara to be applied with a brush or comb.

Thus, for application to the eyelashes or hairs, the composition to which the invention applies can be provided in the form of a pigmented or nonpigmented mascara, to be applied with a brush to the eyelashes or alternatively to the beard or moustache hairs.

For use by injection, the composition can be provided in the form of an aqueous lotion or an oily suspension, for example in the serum form. For use by ingestion or the oral route, it can be provided in the form of capsules, of granules, of syrups to be taken orally or of tablets.

According to a preferred embodiment, the cosmetic composition is provided in the form of a hair cream or lotion, a shampoo, a hair conditioner, a scalp hair mascara or a mascara for the eyelashes. For hair application, the cosmetic composition is preferably a purely aqueous, or aqueous/alcoholic solution or suspension and more preferably a water/ethanol solution or suspension. The alcohol fraction can represent, for example, from 8% to 80%.

Hair is preferably human hair and comprises scalp hair, eyebrows, eyelashes, beard hairs, moustache hairs and pubic hairs. Hair is most preferably human scalp hair. Likewise skin is preferably human skin and most preferably human scalp.

Stems cell are undifferentiated biological cells, that can differentiate into specialized cells and can divide (through mitosis) to produce more stem cells. In mammals, there are two broad types of stem cells: embryonic stem cells, which are isolated from the inner cell mass of blastocysts, and adult stem cells, which are found in various tissues. In adult organisms, stem cells and progenitor cells act as a repair system for the body, replenishing adult tissues. In a developing embryo, stem cells can differentiate into all the specialized cells - ectoderm, endoderm and mesoderm (see induced pluripotent stem cells) - but also maintain the normal turnover of regenerative organs, such as blood, skin, or intestinal tissues. There are three major sources of autologous adult stem cells in humans: (i) Bone marrow, which requires extraction by harvesting, that is, drilling into bone (typically the femur or iliac crest), (ii) Adipose tissue (lipid cells), which requires extraction by liposuction, and (iii) Blood, which requires extraction through pheresis, wherein blood is drawn from the donor (similar to a blood donation), passed through a machine that extracts the stem cells and returns other portions of the blood to the donor.

The embryonic stem cells are preferably from preexisting embryonic stem cell lines, such as those described in Thomson et al (1998), Science Nov 6;282(5391):1145-7. It is also preferred that the embryonic stem cells were obtained from the embryo by means which do not require the destruction of the embryo. Such means are available in the prior art and described, for example, in Chang et al (2008) Cell Stem Cell 7;2(2):113-7.

In accordance with the present invention the stem cells are preferably adult stem cells and more preferably autologous adult stem cells. Of all stem cell types, autologous harvesting involves the least risk of unwanted side effect. By definition, autologous cells are obtained from the same body which receives the cosmetic or pharmaceutical composition described herein.

In the present invention the stem cells are more preferably taken from umbilical cord blood just after birth. Most preferably human umbilical cord blood derived mesenchymal stem cells (hUCBMSCs) are used in accordance with the invention.

The present invention thus also relates to the use of an aqueous cosmetic composition comprising the medium supernatant of a hUCBMSCs culture for caring hair or skin.

Umbilical Cord Blood (UCB) contains a rich source of hematopoietic stem and progenitor cells as well as non-hematopoietic stem cells including endothelial cells, mesenchymal stem cells (MSCs) and unrestricted somatic stem cells (USSC). There are many advantages of UCB as a source of Human Stem Cells (HSCs) as compared to other sources. First, the collection of cord blood units is easy and non-invasive for the donor. Second, the stem cells from UCB can be obtained without ethical consideration. Third, cord blood units could be stored in advance and are therefore rapidly available when needed while bone marrow has to be collected from the donor just before transplantation and there is always a risk of last minute consent refusal. Fourth, cells from UCB can be used directly after isolation without expansion. Finally, the human leukocyte antigen type (HLA) does not need to be matched in case of allogeneic cord blood cell transplantation because these cells are less likely to induce immunological reactions.

The umbilical cord (also called the birth cord or funiculus umbilicalis) is a conduit between the developing embryo or fetus and the placenta. During prenatal development, the umbilical cord is physiologically and genetically part of the fetus and (in humans) normally contains two arteries (the umbilical arteries) and one vein (the umbilical vein), buried within Wharton's jelly. The umbilical vein supplies the fetus with oxygenated, nutrient-rich blood from the placenta. Conversely, the fetal heart pumps deoxygenated, nutrient-depleted blood through the umbilical arteries back to the placenta. The Wharton's jelly is a gelatinous substance made largely from mucopolysaccharides. It contains (in humans) one vein, which carries oxygenated, nutrient-rich blood to the fetus, and two arteries that carry deoxygenated, nutrient-depleted blood away.

In absence of external interventions, the umbilical cord occludes physiologically shortly after birth, explained both by a swelling and collapse of Wharton's jelly in response to a reduction in temperature and by vasoconstriction of the blood vessels by smooth muscle contraction. In effect, a natural clamp is created, halting the flow of blood. In air at 18°C, this physiological clamping will take three minutes or less (Cohain, J. S. (2010). "A Proposed Protocol for Third Stage Management - Judy's 3,4,5,10 minute method". Birth 37 (1): 84-85). General hospital-based obstetric practice introduces artificial clamping as early as 1 minute after the birth of the child. It has to be understood that in the context of the present invention the UCBMSCs are obtained from an umbilical cord after birth and from an umbilical cord which is physiologically occluded (either naturally or via clamp) from the fetus, preferably cut off the fetus.

In more detail, the umbilical cord blood MSCs preferably used in accordance with the invention are obtained from the blood of the umbilical cord. In other terms, first the blood is obtained from the umbilical cord and then the UCBMSCs are obtained from the umbilical cord blood. Umbilical cord blood is a sample of blood taken from a newborn baby's umbilical cord. It is a rich source of stem cells, which to date has been used in the treatment of over 75 diseases, including leukemia, lymphoma and anemia. Parents may choose to bank their newborn's cord blood against the possibility that it will be useful in the future, should the child or a related family member fall victim to a disease that is treatable by cord blood stem cells (Cairo and Wagner (1997), Blood 90 (12): 4665-4678). Cord blood is obtained by syringing out the placenta through the umbilical cord at the time of childbirth, in general after the cord has been detached from the newborn. Cord blood is collected because it contains stem cells, including hematopoietic cells and mesenchymal stem cells, which can be used to treat hematopoietic and genetic disorders. One unit of cord blood generally usually provides stem cells in a quantity sufficient to treat an adult patient. In the United States, the Food and Drug Administration regulates cord blood under the category of "Human Cells, Tissues, and Cellular and Tissue Based-Products." The Code of Federal Regulations under which the FDA regulates public and private cord blood banks is Title 21 Section 1271. Both public and private cord blood banks are eligible for voluntary accreditation with either the American Association of Blood Banks (AABB) or the Foundation for the Accreditation of Cellular Therapy (FACT).

There are several methods for collecting cord blood. The method most commonly used in clinical practice is the "closed technique", which is similar to standard blood collection techniques. With this method, the technician cannulates the vein of the severed umbilical cord using a needle that is connected to a blood bag, and cord blood flows through the needle into the bag. On average, the closed technique enables collection of about 75 ml of cord blood (Christopher D. Hillyer, Ronald G. Strauss & Naomi L. C. Luban (2004), Handbook of Pediatric Transfusion Medicine. Academic Press. pp. 295, 296). Collected cord blood can be cryopreserved and then stored for future transplantation.

As discussed above, cord blood is a rich source of mesenchymal stem cells (MSC). The International Society of Cellular Therapy (ISCT) has established criteria for defining MSC (Dominici et al. (2006). "Minimal criteria for defining multipotent mesenchymal stromal cells. The International Society for Cellular Therapy position statement." Cytotherapy. 8: 315-317). First, MSC must be plastic-adherent when maintained in standard culture conditions. Second, MSC must express CD105, CD73 and CD90, and lack expression of CD45, CD34, CD14 or CD11 b, CD79alpha or CD19 and HLA-DR surface molecules. Third, MSC must differentiate into osteoblasts, adipocytes and chondroblasts in vitro. These criteria may require modification as new knowledge unfolds. It is, though, currently believed that this minimal set of standard criteria fosters a more uniform characterization of MSC. Harvesting the tissue of the umbilical cord can yield between 21 and 500 million MSC (Schugar et al (2009), J Biomed Biotechnol, 2009:789526, doi: 10.1155/2009/7895269). Umbilical cord blood (UCB)-derived mesenchymal stem cells (MSCs) are self-renewing multipotent progenitors with the potential to differentiate into multiple lineages of mesoderm, in addition to generating ectodermal and endodermal lineages by crossing the germline barrier. The collection of mesenchymal stem cells (MSCs) from UCB is a well-established, easy, non-expensive and non-invasive method to collect MSCs (Chang et al. (2006), Cell Biol Int, 30:495-499).

Stem cell culture designates stem cells in a stem cell growth medium (or also named stem cell culture medium). A stem cell growth medium is a liquid (or less preferred a gel) designed to support the growth of the stem cells. The composition of various stem cell growth media are known in the art and these media may either be composed or commercially ordered. Umbilical cord blood derived mesenchymal stem cells (UCBMSCs) are in general cultured in the absence of a feeder layer.

The medium supernatant of a stem cell culture is the stem cell growth medium that covers the cells. The supernatant may be separated from the cells, for example, by a pipette, a centrifuge or a filter. These and other methods are known to the skilled person.

During cell culture the stem cells release factors, in particular cell growth factors into the culture medium. These cell growth factors, such as cytokines, stimulate the cell growth of the stem in the culture. It has to be understood that supernatant comprises in accordance with the present invention these released factors, in particular the cell growth factors in an amount which is sufficient to stimulate cell growth of stem cells.

Also the hair follicle comprises stem cells. In more detail, these stem cells reside in the bulbar region of hair follicles. Stem cells were also detected in the bulge area, a contiguous part of outer root sheath, that provides the insertion point for arrector pili muscle and marks the bottom of the permanent portion of hair follicles (see Ohyama (2007), Journal of Dermatological Science 46:81-89). The stem cells of the hair are keratinocyte stem cells. The hair cycle has been described in detail herein above and therefore the hair stem cell are normally slow cycling, and in case of a constant hair loss slow further down or even stop to cycle. Taylor et al. (2000) Cell, 102:451-61 demonstrated that the bulge stem cells were able to give rise not only to hair follicles but also the epidermis. Consequently, the stem cells in the hair follicle also contribute to build up new skin.

The present invention thus also relates to the use of an aqueous cosmetic composition comprising the medium supernatant of a hair follicle stem cell culture for caring hair or skin.

As mentioned, the skin constantly renews itself throughout adult life, and the hair follicle undergoes a perpetual cycle of growth and degeneration. Stem cells (SCs) residing in the epidermis and, as mentioned, in the hair follicle ensure the maintenance of adult skin homeostasis and hair regeneration, but they also participate in the repair of the epidermis after injuries (see Blanpain and Fuchs (2006), Annu Rev Cell Dev Biol.; 22:339-73). In the adult, many different types of stem cells (SCs) function to replenish these various cell types in skin as it undergoes normal homeostasis or wound repair. For example, also melanoblasts and epidermal SCs reside within the skin itself.

Therefore, a cosmetic composition comprising the medium supernatant of a stem cell culture with the growth factors may be used for caring hair or skin. For example, such use of the cosmetic composition will enhance cell survival of the hair stem cells and/or skin stem cells, turn hair stem cells and/or skin stem cells from a quiescent to a proliferative state, and/or repair damaged hair stem cells and/or skin stem cells. Likewise and as further detailed in the following, a pharmaceutical composition comprising the medium supernatant of a stem cell culture with the growth factors may be used for the prevention or treatment of alopecia. Also the pharmaceutical composition will enhance cell survival of the hair stem cells, turn hair stem cells from a quiescent to a proliferative state, and/or repair damaged hair stem cells.

In a second aspect the present invention relates to an aqueous pharmaceutical composition comprising the medium supernatant of a stem cell culture for use in the prevention or treatment of alopecia.

The present invention further relates to an aqueous pharmaceutical composition comprising the medium supernatant of a hUCBMSCs culture for use in the prevention or treatment of alopecia.

The present invention further relates to an aqueous pharmaceutical composition comprising the medium supernatant of a hair follicle stem cell culture for use in the prevention or treatment of alopecia.

It has to be understood that the medium supernatant of a stem cell culture is used in an amount which effective for the prevention or treatment of alopecia. The supernatant of a stem cell culture may either be directly used for the preparation of a cosmetic composition or further processed before use, such as fractioned or purified.

Alopecia is the medical term for hair loss. Symptoms of alopecia may not only include hair loss, but also, for example, skin lesions, and scarring. There are many types of hair loss with different symptoms and causes. Further details on the types of alopecia are provided herein below.

The prevention or treatment of alopecia involves bringing the skin, preferably the scalp into contact with the pharmaceutical composition. Consequently, the invention also pertains to an aqueous pharmaceutical composition which comprises the medium supernatant of a stem cell culture.

An aqueous pharmaceutical composition is a pharmaceutical composition in which one, preferably the main solvent is water. The aqueous phase thus comprises water and may optionally comprise an ingredient miscible in any proportion with water, such as lower C₁ to C₈ alcohols, for example ethanol or isopropanol, polyols, such as propylene glycol, glycerol or sorbitol, or else acetone or ether.

The pharmaceutical composition for use in accordance with the present invention can be formulated in conventional manner according to methods found in the art, using one or more physiological carriers or excipient, see, for example Ansel et al., "Pharmaceutical Dosage Forms and Drug Delivery Systems", 7th edition, Lippincott Williams & Wilkins Publishers, 1999. The pharmaceutical composition may, accordingly, be administered locally or topically e.g. via iontopheresis, systemically, orally, parenterally, such as subcutaneously, intravenously, intramuscularly, intraperitoneally, intrathecally, transdermally, transmucosally, subdurally, sublingually, by inhalation spray, aerosol or rectally and the like in dosage unit formulations optionally comprising conventional pharmaceutically acceptable excipients.

Pharmaceutical compositions of the invention preferably comprise a pharmaceutically acceptable carrier. By "pharmaceutically acceptable carrier" is meant a non-toxic solid, semisolid or liquid filler, diluent, encapsulating material or formulation auxiliary of any type.

A preferred pharmaceutical composition is formulated for topical application to the skin, preferably scalp and hair, preferably scalp hair. For topical application to the skin, including the scalp, the composition preferably have the form of an aqueous, or aqueous/alcoholic solution or suspension; an oily suspension or solution; an emulsion (water-in-oil or oil-in-water) or dispersion with a liquid or semi-liquid consistency obtained by dispersion of a fatty phase in an aqueous phase (O/W) or vice versa (W/O); a dispersion or emulsion with a soft consistency; oran aqueous or aqueous/alcoholic gel.. The composition can also have the form of microcapsules or microparticles, or of vesicular dispersions of ionic and/or nonionic type. It is also possible to envisage a composition in the form of a foam or alternatively in the form of an aerosol composition also comprising a pressurized propellant.

A pharmaceutical composition may comprise a patient information leaflet, which is a leaflet containing specific information about medical conditions, doses, side effects that packed with medicines to give the user information about the product.

In particular, the composition for application to the scalp or scalp hair can be provided in the form of a hair care lotion, for example for daily or twice-weekly application, of a shampoo or of a hair conditioner, in particular for twice-weekly or weekly application, of a liquid or solid soap for cleaning the scalp for daily application, of a product for shaping the hairstyle (lacquer, hairsetting product, styling gel), of a treatment mask, of a cream or of a foaming gel for cleaning the hair.

Furthermore, for application to the eyelashes or hairs, the composition to which the invention applies can be provided in the form of a pigmented or nonpigmented mascara, to be applied with a brush to the eyelashes or alternatively to the beard or moustache hairs.

For use by injection, the composition can be provided in the form of an aqueous lotion or an oily suspension, for example in the serum form. For use by ingestion or the oral route, it can be provided in the form of liquid-filled capsules, of liquid-filled granules, of syrups to be taken orally or of tablets.

According to a preferred embodiment, the phramaceutical composition is provided in the form of a hair cream or lotion, of a shampoo, of a hair conditioner, of a hair mascara.. For hair application, the pharmaceutical composition is preferably an aqueous, or aqueous/alcoholic solution or suspension and more preferably a water/ethanol solution or suspension. The alcohol fraction can represent from 5% to 99.9% and preferably from 8% to 80%.

According to a preferred embodiment of the first and second aspect of the invention, the (cosmetic or pharmaceutical) composition further comprises epidermal growth factor.

Epidermal growth factor (EGF) is a growth factor that stimulates cell growth, proliferation, and differentiation by binding to its receptor EGFR. Human EGF is a 6.2kDa protein with 53 amino acid residues and three intramolecular disulfide bonds. Epidermal growth factor is used in the art as anti-wrinkle and skin-lifting agent. In more detail, it reduces and prevents lines and wrinkles by actively generating new skin cells; enhances tone of skin that brimming with vitality and energy; nourishes skin that appears smoother, brighter and regains youth look; and most importantly eliminates scars of face by forming new skin and hair cells. EGF is preferably recombinantly produced EGF. EGF may be recombinantly produced in a bacterial host cells, such an *E. coli* cell.

EGF is used with increasing preference at 0.1 to 5 %, 0.2 to 4%, 0.3 to 3%, 0.5 to 1.5 %, and about 1% of the total weight of the composition. EGF preferably has concentration of about 10ppm in the composition.

The present invention thus also relates to the use of an aqueous cosmetic composition comprising the medium supernatant of a hUCBMSCs culture for caring hair or skin, wherein the composition further comprises epidermal growth factor. The present invention further relates to an aqueous pharmaceutical composition comprising the medium supernatant of a hUCBMSCs stem cell culture for use in the prevention or treatment of alopecia, wherein the composition further comprises epidermal growth factor.

In accordance with the first and second aspect of the invention, the (cosmetic or pharmaceutical) composition preferably further comprises one or more biomimetic peptide(s).

Biomimetic peptides mimic the function of growth factors as messengers in the skin, allowing the epidermis and dermis to communicate more efficiently. This is important, because one of the key effects of aging is reduced communication within the skin, resulting in decreased collagen production and an aggregation of degraded elastin fibers. Decreased collagen production (less supportive, firming fibres), coupled with degraded elastin (diminished skin elasticity and flexibility) combine exponentially to produce classic visible signs of aging; including thinner skin, fine and deep wrinkles, sagging and flaccidity and overall loss of skin tone.

The biomimetic peptides are preferably recombinantly produced human growth factors One example and the most preferred biomimetic peptide is the above-described recombinant EGF. Further non-limiting examples of biomimetic peptides are recombinantly produced acidic fibroblast growth factor, basic fibroblast growth factor, insulin growth factor 1, insulin growth factor 2, vascular endothelial growth factor, thymosin ß4, thioreducin, keratinocyte growth factor, transforming growth factor ß1, transforming growth factor ß3 and transforming growth factor α.

In a preferred embodiment of the first and second aspect of the invention, the aqueous phase of the (cosmetic or pharmaceutical) composition consists with increasing preference of at least 0.05%, at least 0.1%, at least 0.2%, at least 0.3%, at least 0.4%, at least 0.5%, at least 0.1%, at least 0.15%, at least 0.2%, at least 0.25%, at least 0.3%, at least 0.4%, at least 0.5%, at least 0.6%, at least 0.7%, at least 0.8%, at least 0.9%, at least 1.0%, at least 2.0%, at least 3.0%, at least 4.0%, at least 5.0%, at least 7.5%, at least 10.0%, at least 15.0%, at least 20.0%, at least 25.0%, at least 30.05%, at least 40.0%, at least 50.0%, at least 60.0%, at least 70.0%, at least 80.0%, at least 90.0%, at least 95.0%, at least 97.5%, at least 98.0%, at least 99.0%, at least 99.5%, at least 99.6%, at least 99.7%, at least 99.8%, at least 99.0%, 100% medium supernatant by total weight (w/w) of the aqueous phase.

It has to be understood that it's a matter of routine for the skilled person to determine an amount of medium supernatant which is sufficient to bring about the desired technical effect when the cosmetic or pharmaceutical composition of the invention is applied.

The desired technical effect of the cosmetic composition is the caring of hair or skin. Hair is cared, for example, if dandruffs are reduced, the hair is strengthened or detangled, the hair volume or vigor is increased, hair shine, bounce, or feel (such as softness) is improved. Skin is cared, for example, if the oily shine, roughness, flaking, itching, burning, reddening, pimples, blisters of the skin is reduced; or tightness (i.e. less wrinkles), texture, tone or dryness of the skin is improved.

The desired technical effect of the cosmetic composition is the prevention or treatment of alopecia. As also discussed herein above, natural hair loss (i.e. shedding) can be estimated, on average, at a few hundred individual hairs per day for a normal physiological state. Humans normally shed, for example, 50-100 scalp hairs. These hairs are normally replaced by new hairs. Hence, hair loss is prevented if the number of hair remains substantially constant. As also discussed above, bald patches or lots of thinning of hair indicate an abnormal hair loss.

Also depending on the particular formulation of the composition the portion of the aqueous phase may vary and consequently also the percentage medium supernatant by total weight (w/w) of the aqueous phase. The compositions may also comprise a non-aqueous phase, such as an oily phase. The compositions may be, for example, water in oil or oil in water emulsions. The smaller the weight-% of aqueous phase is in the composition the higher is the weight-% of medium supernatant by total weight (w/w) of the aqueous phase. The total weight (w/w) of the medium supernatant in the final composition is with increasing preference 0.5 to 15%, 1.0 to 10%, 1.0 to 5%, 1.5 to 3%, and about 2% by total weight of the final composition.

The present invention thus also relates to the use of an aqueous cosmetic composition comprising the medium supernatant of a hUCBMSCs culture in amount of about 2% by total weight of the final composition for caring hair or skin, wherein the composition further comprises epidermal growth factor. The present invention further relates to an aqueous pharmaceutical composition comprising the medium supernatant of a hUCBMSCs stem cell culture in amount of about 2% by total weight of the final composition for use in the prevention or treatment of alopecia, wherein the composition further comprises epidermal growth factor.

In a preferred embodiment of the first and second aspect of the invention, the (cosmetic or pharmaceutical) composition is stem cell-free and preferably cell-free.

Residual amounts of stem cells in the supernatant may cause unwanted side effects, such as an inflammatory response. Thus, the composition is preferably stem cell-free. As known in the art, stem cells may be grown on a feeder cell layer. Hence, a stem cell culture may also comprise cells others than stem cells. Consequently, the (cosmetic or pharmaceutical) composition is preferably cell-free. The cells can be separated from the medium by methods well-known in the art, such a centrifugation or filtration.

In a preferred embodiment of the first and second aspect of the invention, the medium supernatant is from a subconfluent stem cell culture.

A subconfluent stem cell culture is a stem cell culture in which the spatial relationships of the cells are intermediate between sparse and confluent. In a subconfluent stem cell culture preferably 50-95%, and more preferably 75%-90% of the growth surface is covered by stem cells.

Also in a stem cell culture used in accordance with the cell density is with increasing preference 1x10⁴ to 1x10⁸ per ml of culture medium, 1x10⁵ to 1x10⁷ per ml of culture medium, 5x10⁵ to 5x10⁶ per ml of culture medium, 8x10⁵ to 3x10⁶ per ml of culture medium, 9x10⁵ to 2x10⁶ per ml of culture medium, and about 1x10⁶ per ml of culture medium.

The temperature in the stem cell culture is with increasing preference 35 to 39°C, 36 to 38°C, 36.5 to 37.5°C, and about 37°C.

The cells are preferably cultured in T75 tissue flask which has a cell growth area of 75cm².

E.g. in case of using a T75 tissue flask, the total medium volume used in the stem cell culture is with increasing preference 10-20mL, 12.5-17.5mL, 14-16ml, and about 15mL.

The CO₂ content is with increasing preference 2.5-7.5%, 4-6%, and about 5%.

The above characteristics are of course combinable. Hence, the subconfluent stem cell culture has, for example, the following characteristics: cell density of 9x10⁵ to 2x10⁶ per ml of culture medium; culture temperature of 36.5 to 37.5°C; medium volume of 14-16ml, CO₂ content of 4-6%, and CO₂ content of 4-6%.

In another preferred embodiment of the first and second aspect of the invention, the medium supernatant is from a stem cell culture, wherein the stem cells have been cultured in the medium with increasing preference for at least 4 hours (h), at least 8h, at least 12h, at least 16h, at least 24h, at least 36h, at least 48h, at least 60h, at least 72h, at least 84h, and at least 96h.

As discussed herein above, the stem cells release factors during cell culture into the supernatant of the culture medium which trigger the desired technical effect of the cosmetic or pharmaceutical composition of the invention. It is a routine work for the skilled person to culture the cells for a time which allows that the cells produce a concentration of the stem cell-released factors within the culture medium which allows caring of hair and skin in the context of the cosmetic composition; or treating or prevention of alopecia in the context of the pharmaceutical composition.

According to a further preferred embodiment of the first and second aspect of the invention, the stem cells are mammalian stem cells, preferably primate stem cells, more preferably human stem cells, and most preferably human umbilical cord blood derived mesenchymal stem cells.

In accordance with this preferred embodiment also the individual which receives the cosmetic or pharmaceutical composition of the invention is a mammal, a preferably a primate and more preferably human. As also discussed above, the stem cells are preferably adult stem cells, and more preferably adult autologous stem cells.

Is has to be understood that the subject to which the cosmetic or pharmaceutical composition is applied is preferably of the same species and the species from which the stem cells were obtained. Most preferred this species is *Homo sapiens.*

In a further preferred embodiment of the first and second aspect of the invention, the medium is Dulbecco's Modified Eagle Medium (DMEM) or modified DMEM.

DMEM is a frequently used cell culture medium, because DMEM is suitable for most types of cells, including human, monkey, hamster, rat, mouse, chicken and fish. The medium comprises in general amino acids, salts (calcium chloride, potassium chloride, magnesium sulfate, sodium chloride, and monosodium phosphate), glucose, vitamins (folic acid, nicotinamide, riboflavin, B₁₂), iron and phenol, red.

Preferably a low glucose DMEM, which is DMEM having 1.0 g/L Glucose is used. Low glucose DEMEM is commercially availabe, for example, from Invitrogen or Lifetech.

The present invention thus also relates to the use of an aqueous cosmetic composition comprising the medium supernatant of a hUCBMSCs culture in amount of about 2% by total weight of the final composition for caring hair or skin, wherein the composition further comprises epidermal growth factor, and wherein the medium is low glucose DMEM. The present invention further relates to an aqueous pharmaceutical composition comprising the medium supernatant of a hUCBMSCs stem cell culture in amount of about 2% by total weight of the final composition for use in the prevention or treatment of alopecia, wherein the composition further comprises epidermal growth factor, and wherein the medium is low glucose DMEM.

The present invention furthermore also relates to the use of an aqueous cosmetic composition comprising the medium supernatant of a hUCBMSCs culture a cell-density of about 1x10⁶ per ml in amount of about 2% by total weight of the final composition for caring hair or skin, wherein the composition further comprises epidermal growth factor, and wherein the medium is low glucose DMEM. The present invention further relates to an aqueous pharmaceutical composition comprising the medium supernatant of a hUCBMSCs stem cell culture having a cell-density of about 1x10⁶ per ml in amount of about 2% by total weight of the final composition for use in the prevention or treatment of alopecia, wherein the composition further comprises epidermal growth factor, and wherein the medium is low glucose DMEM.

In a more preferred embodiment of the first and second aspect of the invention, the DMEM or modified DMEM is selected from the group consisting of media having ATCC Number 30-2002, 30-2601 and 30-2006.

In a different preferred embodiment of the first and second aspect of the invention, the composition comprises a consistency-giving component selected from the group consisting of poly(acrylic acid), xanthan gum, pectin or a combination thereof, wherein the consistency-giving component is preferably present at 0.001 to 10%, more preferably 0.005 to 5%, even more preferably 0.01 to 2%, further more preferably 0.05 to 1.5% and most preferably 0.1 to 1% by total weight (w/w) of the composition.

A consistency-giving component determines the appearance of the pharmaceutical and cosmetic compositions of the invention. For example, poly(acrylic acid)s are frequently used as a thickening, dispersing, suspending and emulsifying agents in pharmaceutical and cosmetic compositions. Xanthan gum is a polysaccharide secreted by the bacterium *Xanthomonas campestris,* which may be used as rheology modifier, thickening agent and stabilizer (for example, to prevent ingredients from separating). Pectin is used for example as gelling agent.

In a preferred embodiment of the first and second aspect of the invention, the composition comprises a surfactant, wherein the surfactant is preferably present at 0.001 to 10%, more preferably 0.005 to 5%, even more preferably 0.01 to 2%, further more preferably 0.05 to 1.5% and most preferably 0.1 to 1% by total weight (w/w) of the composition.

Surfactants are compounds that lower the surface tension (or interfacial tension) between two liquids or between a liquid and a solid. Surfactants may act as detergents, wetting agents, emulsifiers, foaming agents, and dispersants. Surfactants are usually organic compounds that are amphiphilic, meaning they contain both hydrophobic groups (their tails) and hydrophilic groups (their heads).

Non-limiting examples of surfactants which may be used in accordance with the invention are sodium cocoyl alaninate, decyl glucoside, cocamidopropyl betaine, coco glucoside, sodium lauryl aulfate, cocamidopropyl betaine, sodium laureth sulfate, stearamidopropyl dimethylamine, sodium laureth-5 carboxylate , laureth-11 carboxylic acid, sodium lauroyl sarcosinate, disodium cocoyl glutamate" sodium Cocoyl glutamate, disodium cocoyl-glutamate, sodium cocoyl isethionate, sodium coco-glucoside tartrate, sodium coco sulfate, polyglyceryl-10 laurate, sodium cocoyl glutamate, sodium lauroyl sarcosinate, sodium capryloyl glutamate, cocamidopropyl betaine, disodium cocoamphodiacetate, cocamidopropyl betaine, TIPA lauryl sulfate, sodium lauryl sulfate, sodium myristyl sulfate, sodium stearyl sulfate, laureth-10 and sorbitan oleate.

In a further preferred embodiment of the first and second aspect of the invention, the aqueous phase represents 15 to 99%, preferably 30 to 98%, more preferably 30 to 97%, and most preferably 70 to 95% by total weight (w/w) of the composition.

As discussed above, the compositions described herein are aqueous. However, the compositions may also comprise a non-aqueous phase, such as an oily phase. The compositions may be, for example, water in oil or oil in water emulsions. Depending on the particular formulation the portion of the aqueous phase may vary within the above-indicated preferred ranges.

In accordance with a further preferred embodiment of the first and second aspect of the invention, the composition comprises a preservative agent, wherein the preservative agent is preferably present at 0.001 to 10%, more preferably 0.005 to 5%, even more preferably 0.01 to 2%, further more preferably 0.05 to 1.5% and most preferably 0.1 to 1% by total weight (w/w) of the composition.

Preservative agents prevent that the compositions described herein become contaminated, for example, by bacteria and fungi. Preservative agents thus comprise anti-microbial agents. Common anti-microbial preservatives include sorbic acid and its salts, benzoic acid and its salts, calcium propionate, sodium nitrite (and sodium nitrate which converts to sodium nitrite "in situ"), sulfites (sulfur dioxide, sodium bisulfite, potassium hydrogen sulfite, etc.) and disodium EDTA. Preservative agents can also be antioxidans such as oxygen absorbers, which inhibit the oxidation of the compositions described herein. Antioxidants include BHA, BHT, TBHQ and propyl gallate. Other preservatives include ethanol and methylchloroisothiazolinone.

A preservative may be a naturally occurring or synthetically produced substance. Preservatives can be divided into two types, depending on their origin. Class I preservatives refers to those preservatives which are naturally occurring, everyday substances. Examples include salt, honey and wood smoke. Class II preservatives refer to preservatives which are synthetically manufactured.

In another preferred embodiment of the first and second aspect of the invention, the composition comprises a pH buffer, wherein the pH buffer is preferably present at 0,001 to 10%, more preferably 0.005 to 5%, even more preferably 0.0008 to 2%, more preferably 0.01 to 1 % and most preferably 0.1 to 1 % by total weight (w/w) of the composition.

A pH buffer prevents changes in the pH in the compositions described herein. In general, a pH buffer is a weak acid or base used to maintain the acidity (pH) of a solution near a chosen value after the addition of another acid or base. That is, the function of a buffering agent is to prevent a rapid change in pH when acids or bases are added to the solution. Buffering agents have variable properties. Some are more soluble than others; some are acidic while others are basic.

Suitable buffers pH buffer are known to the skilled person and comprise a phosphate buffer, a citric acid buffer or a carbonate buffer. Preferred pH buffers which are used in accordance with the invention are sodium hydrogen sulfate/sodium sulfate; sodium dihydrogen sulfate/sodium hydrogensulfate; and potassium hydrogensulfate/potassium sulfate.

In still further preferred embodiment of the first and second aspect of the invention, in the aqueous phase of the composition 25 to 50 ml/l, preferably 27,5 to 47.5 ml/l, more preferably 30 to 45 ml/l, even more preferably 32,5 to 43.5 ml/l and most preferably 39 to 40 ml/l nitrogen is solved.

Nitrogen (in particular NO gas) can be used as propellant in a composition described herein. Accordingly, the composition comprising nitriogen is preferably a spray, such as hair spray, a foam or an aerosol.

The cosmetic and pharmaceutical compositions according to the first and second aspect of the invention may comprise, for example, the following further ingredients (or any combination thereof), wherein some of these classes have been mentioned above and wherein some of these classes may not be present in the cosmetic compositions: (i) Hormones, in particular of plant origin, for example estriol or its analogues, thyroxine and its salts, or progesterone; (ii) antibacterial agents, such as macrolides, pyranosides and tetracyclines and in particular erythromycin; (iii) calcium antagonist agents, such as cinnarizine, diltiazem, nimodipine, verapamil and nifedipine; (iv) microorganism extracts, in particular bacterial extracts; (v) agents which modulate cutaneous differentiation and/or proliferation, such as retinoic acid and its isomers, retinol and its esters, vitamin D and its derivatives, or oestrogens, such as oestradiol; (vi) agents which modulate bacterial adhesion to the skin and/or mucous membranes, such as honey, in particular acacia honey, and certain sugar derivatives; (vii) agents for combating parasites, in particular metronidazole, crotamiton or pyrethroids; (viii) antifungals, in particular compounds belonging to the imidazole class, such as econazole, ketoconazole or miconazole or their salts, nicotinic acid esters, including in particular tocopherol nicotinate, benzyl nicotinate and C₁-C₆ alkyl nicotinates, such as methyl nicotinate or hexyl nicotinate; polyene compounds, such as amphotericin B, compounds of the allylamine family, such as terbinafine, or alternatively octopirox, selenium derivatives or anthralin; (ix) antiviral agents, such as acyclovir; (x) steroidal anti-inflammatory agents, such as cortico-steroids, for example hydrocortisone, betamethasone valerate or clobetasol propionate, or nonsteroidal anti-inflammatory agents, such as ibuprofen and its salts, diclofenac and its salts, acetylsalicylic acid, acetaminophen, glycyrrhetinic acid or .alpha.-bisabolol; (xi) anaesthetic agents, such as lidocaine hydrochloride and its derivatives; (xii) antipruriginous agents, such as thenaldine, trimeprazine or cyproheptadine; (xiii) keratolytic agents, such as .α- and β-hydroxy-carboxylic or β-ketocarboxylic acids, their salts, amides or esters, the lactones and their corresponding salts, and more particularly hydroxy acids, such as glycolic acid, lactic acid, salicylic acid, citric acid and generally fruit acids, or salicylic acid derivatives, such as those carrying an alkanoyl radical having from 2 to 12 carbon atoms in the 5 position of the benzene ring, such as 5-(n-octanoyl)salicylic acid; (xiv) agents for combating free radicals, such as .alpha.-tocopherol or its esters, superoxide dismutases, certain metal chelating agents or ascorbic acid and its esters; (xv) antiseborrheics, such as progesterone; (xvi) antidandruff agents, such as octopirox or zinc pyrithione; (xvi) anti-acne agents, such as retinoic acid or benzoyl peroxide; (xvii) vasodilators, such as pyrimidine derivatives, for example 2,4-diamino-6-piperidinopyrimidine 3-oxide or "minoxidil", described in US 4,139,619 and US 4,596,812, or diazoxide; (xviii) agents which reduce hair loss, such as aminexil or 6-O-[(9Z,12Z)-octadeca-9,12-dienoyl]hexapyranose; (xix) anti-androgen agents, such as oxendolone, spironolactone, diethylstilbestrol and flutamide; (xx) steroidal or nonsteroidal inhibitors of 5.alpha.-reductases, such as finasteride; (xxi) potassium channel agonists, such as cromakalim and nicorandil; and (xxii) FP receptor (receptor to prostaglandins of type F) agonists, such as latanoprost, bimatoprost, travoprost or unoprostone.

As mentioned, the composition according to the invention may in particular comprise at least one additional active principle which promotes hair or skin, such as aminexil, minoxidil, latanoprost and/or travoprost.

In a third aspect the present invention relates to a method for caring hair or skin comprising bringing into contact the cosmetic composition of the invention (which is as defined in connection with the first aspect of the invention) with skin or the scalp for a time which is sufficient to care the hair or skin.

Indications and parameters of caring hair or skin have been discussed above. Based on these indications and parameters the skilled person can determine a time which is sufficient to care the hair or skin. The time which is sufficient to care the hair or skin may be between 1 min and 24 hours, and more preferred between 1 hour and 12 hours.

The method preferably comprises the additional step of washing-off the cosmetic composition after the time which is sufficient to care the hair or skin.

In a preferred embodiment of the first and third aspect of the invention, hair is cared by (i.e. the technical effect of caring hair is) (a) making up the keratinous fibers of hair, preferably human hair, (b) inducing or stimulating the growth of the keratinous fibers of hair, preferably human hair, (c) slowing down or preventing the loss of the keratinous fibers of hair, preferably human hair, (d) increasing the density of the keratinous fibers of hair, preferably human hair; and/or (e) increasing or maintaining the viability of keratinocytes, in particular of hair follicle kerationcytes.

The keratinous fibers are preferably those of the scalp hair, eyebrows, eyelashes, beard hairs, moustache hairs and pubic hairs, and more preferably the scalp hair and/or eyelashes.

In another preferred embodiment of the first and third aspect of the invention, skin is cared by (i.e. the technical effect of caring skin is) (a) improving skin appearance, in particular scalp appearance, and/or (b) reducing skin aging.

In a preferred embodiment of the second aspect of the invention, the alopecia is androgenetic alopecia, alopecia areata, alopecia mucinosa, diffuse alopecia, alopecia actinica, alopecia mechanis, alopecia seborrhoica, alopecia parvimaculata, alopecia senilis, alopecia syphilitica, alopecia triangularis congenita Sabouraud, or alopecia congenita.

Androgenetic alopecia is hair loss that occurs due to an underlying susceptibility of hair follicles to androgenic miniaturization. It is the most common cause of hair loss and will affect up to 70% of men and 40% of women at some point in their lifetime. Therefore, androgenetic alopecia is the most preferred alopecia to be prevented or treated in accordance with the presented invention.

Alopecia areata typically presents with sudden hair loss causing patches to appear on the scalp or other areas of the body. If left untreated, or if the condition does not respond to treatment, complete baldness can result in the affected area, which is referred to as alopecia totalis. When the entire body suffers from complete hair loss, it is referred to as alopecia universalis.

Alopecia mucinosa is often also referred to as follicular mucinosis. The dermatologic eruptions consist of follicular papules and/or indurated plaques that demonstrate distinct histologic changes in the hair follicles that lead to hair loss. The accumulation of mucinous material in the damaged hair follicles and sebaceous glands creates an inflammatory condition and subsequent degenerative process. The face, the neck, and the scalp are the most frequently affected sites, although lesions may appear on any part of the body.

Diffuse alopecia appears in both women and men; however, women are much more affected than men. Diffuse hair loss can occur at any age and is characterized by thinning of the entire scalp, followed by thinning of the hair (the hair loses its density). Diffuse hair loss is temporary; hair grows back providing the hair follicle remains active.

Alopecia actinica is caused by radiation, for example, in a radiation therapy to treat cancer.

Alopecia mechanis is caused physical means, such as tension or rubbing.

Alopecia seborrhoic is premature baldness in men. In alopecia seborrhoica hair loss steadily advances from the forehead backwards, until only a fringe of hair is left on the head.

Alopecia parvimaculata, which is typical of childhood, is characterized by small areas of alopecia with inflamed and atrophic skin and irregularly angular, round or oval shaped.

Alopecia senilis is age-related hair loss. Baldness due to old age is seldom seen before the 40th year of age. It usually begins at the vertex and spreads forward and backward until the whole crown is symmetrically affected. It may also be a general thinning of the scalp or it may start in the brow and show its advance by a receding forehead.

Alopecia syphilitica is one of the clinical manifestations of secondary syphilis.

Alopecia triangularis congenita Sabouraud is a type of congenital alopecia occurring on the temples. It is characterized by a unilateral or bilateral patch of complete alopecia of triangular form. Its base is directed toward the forehead, and its longest diameter is 1 to 1½ inches (2.5 to 4 cm.) long.

Alopecia congenita is a rare condition usually caused by a delay in the development of the hair. Heridity is considered to be a causative factor for this condition. Hair may grow late after a week or month, or may not grow in some exceptional cases.

In a further preferred embodiment of the second aspect of the invention, the alopecia is caused by one or more of chronic inflammation, diabetes, dissecting cellulitis, a fungal infections (such as tinea capitis), hyperthyroidism and hypothyroidism, hypervitaminosis A, iron deficiency, lupus erythematosus, side effects from drugs (such as chemotherapy, anabolic steroids, and birth control pills), pseudopelade of brocq, radiation therapy, scalp infection (such as dermatophyte and tinea capitis), secondary syphilis, telogen effluvium, traction alopecia, trichotillomania and tufted folliculitis.

Alopecia is a side effect of many diseases and treatments. The above list of diseases and treatments provides non-limiting examples of diseases and treatments which are associated with alopecia.

It is understood that all these disease can be treated with the pharmaceutical composition of the invention, and that is can be used for their prevention, respectively.

In a fourth aspect the present invention relates to a method of manufacturing a composition as defined in connection with the first or second aspect of the invention, which comprises (a) culturing stem cells in a medium, (b) obtaining the supernatant of the stem cell culture of step (a), (c) filling the the supernatant of a stem cell culture obtained in step (b) and the other ingredients of the composition into a pressure-resistant container, and (d) applying gas to the composition during and/or after step (c).

In a preferred embodiment of the fourth aspect of the invention, the gas is propan, butan, dimethylether or mixes thereof, or NO or NO₂.

Butane (which may be also Isobutane) and Propane are colorless and odorless gases. In cosmetics and personal care products, these ingredients are used in the formulation of shaving cream, cleansing products, hair conditioners, and makeup. Butane, Isobutane and Propane may compressed and used as aerosol propellants.

NO (nitric oxide) or NO₂ (nitrogen dioxide) are approved for use as a food additive (also known as E942), specifically as an aerosol spray propellant. These gases may also be used to displace oxygen, to inhibit bacterial growth.

In another preferred embodiment of the fourth aspect of the invention, the container is protected from light and consists of glass and/or polyethylene terephthalate (PET).

To protect the container from light, for example, brown or green glass may be used. Likewise PET may be colored. The thickness of glass and PET is adjusted such that it is sufficiently pressure-resistant, in particular during step (d) of the method according to the fourth aspect of the invention.

The examples illustrate the invention:

### 1. Exemplary composition

| INGREDIENTS | weight % |
|---|---|
| Aqua | ad 100 |
| Aloe Barbadensis Leaf Juice | 10 |
| Cellculture Solution after treatment with stem cells (Blue horizon) (i.e. medium supernatant of a stem cell culture) | 2,00 |
| Caprylyl/Capryl Wheat Bran/Straw Glycosides, Aqua, Fusel Wheat Bran/Straw Glycosides, Polyglyceryl-5 Oleate, Sodium Cocoyl Glutamate, Glyceryl Caprylate | 0,80 |
| Ceramide-3, Cetyl Palmitate, Glycerin, Laureth-23, | 2,00 |
| Vitis Vinifera (Grape) Vine Extract | 0,01 |
| Hyaluronamid | 0,20 |
| rh-Oligopeptide-1 (i.e. recombinant EGF) | 1,00 |
| Sodium Hyaluronate | 0,10 |
| pH-Regulator (i.e. pH buffer) | 0,30 |
| Parfum | 0,01 |

### 2. DMEM exemplary basic solution (contents in mg/l)

Inorganic salts:
- CaCl₂ (water free): 200.00
- Fe(NO₃)₃ 9 H₂O: 0.10
- KCl:400.00
- MgSO₄ (water free): 97.67
- NaCl: 6400.00
- NaH₂PO₄ · H₂O: 125.00
- NaHCO₃: 3700,00 (3,7g / l = 0.37%)

Other components:
- D-Glucose: 1000.00
- Phenole red: 15.00
- Sodium pyruvate: 110.00

Amino acids:
- L-Arginine HCl: 84.00
- L-Cysteine 2HCl: 63.00
- L-Glutamine:584.00
- Glycin:30.00
- L-Histidine HCl H₂O: 42.00
- L-Isoleucine: 105.00
- L-Leucine: 105.00
- L-Lysin HCl: 146.00
- L-Methionine: 30.00
- L-Phenylalanine: 66.00
- L-Serine: 42.00
- L-Threonine: 95.00
- L-Tryptophan: 16.00
- L-Tyrosine 2Na · 2H₂O: 104,33
- L-Valine: 94.00

Vitamins:
- D-Calciumpantothenate: 4.00
- Choline chloride: 4.00
- Folic acid 4.00
- i-Inosito 17.20
- Niacinamide 4.00
- Riboflavin 0.40
- Thiamine HCl 4.00

## Claims

1. Use of an aqueous cosmetic composition comprising the medium supernatant of a stem cell culture for caring hair or skin.

2. An aqueous pharmaceutical composition comprising the medium supernatant of a stem cell culture for use in the prevention or treatment of alopecia.

3. The use of claim 1 or the pharmaceutical composition for use according to claim 2, wherein the composition further comprises epidermal growth factor.

4. The use of claim 1 or 3, or the pharmaceutical composition for use according to claim 2 or 3, wherein the aqueous phase of the composition consists with increasing preference of at least 0.05%, at least 0.1%, at least 0.2%, at least 0.3%, at least 0.4%, at least 0.5%, at least 0.1%, at least 0.15%, at least 0.2%, at least 0.25%, at least 0.3%, at least 0.4%, at least 0.5%, at least 0.6%, at least 0.7%, at least 0.8%, at least 0.9%, at least 1.0%, at least 2.0%, at least 3.0%, at least 4.0%, at least 5.0%, at least 7.5%, at least 10.0%, at least 15.0%, at least 20.0%, at least 25.0%, at least 30.05%, at least 40.0%, at least 50.0%, at least 60.0%, at least 70.0%, at least 80.0%, at least 90.0%, at least 95.0%, at least 97.5%, at least 98.0%, at least 99.0%, at least 99.5%, at least 99.6%, at least 99.7%, at least 99.8%, at least 99.0%, 100% medium supernatant by total weight (w/w) of the aqueous phase.

5. The use of any one claims 1, 3 and 4, or the pharmaceutical composition for use according to any one of claims 2 to 4, wherein the composition is stem cell-free and preferably cell-free.

6. The use of any one claims 1, and 3 to 5, or the pharmaceutical composition for use according to any one of claims 2 to 5, wherein the medium supernatant is from a subconfluent stem cell culture.

7. The use of any one claims 1, and 3 to 6, or the pharmaceutical composition for use according to any one of claims 2 to 6, wherein the medium supernatant is from a stem cell culture, wherein the stem cells have been cultured in the medium with increasing preference for at least 4h, at least 8h, at least 12h, at least 16h, at least 24h, at least 36h, at least 48h, at least 60h, at least 72h, at least 84h, and at least 96h.

8. The use of any one claims 1, and 3 to 7, or the pharmaceutical composition for use according to any one of claims 2 to 7, wherein the stem cells are mammalian stem cells, preferably primate stem cells, more preferably human stem cells and most preferably human umbilical cord blood derived mesenchymal stem cells.

9. The use of any one claims 1, and 3 to 8, or the pharmaceutical composition for use according to any one of claims 2 to 8, wherein the medium is Dulbecco's Modified Eagle Medium (DMEM) or modified DMEM.

10. The use of claim 9, or the pharmaceutical composition for use according to claim 8, wherein the DMEM or modified DMEM is selected from the group consisting of media having ATCC Number 30-2002, 30-2601 and 30-2006.

11. The use of any one claims 1, and 3 to 10, or the pharmaceutical composition for use according to any one of claims 2 to 10, wherein the composition comprises a consistency-giving component selected from the group consisting of poly(acrylic acid), xanthan gum, pectin or a combination thereof, wherein the consistency-giving component is preferably present at 0.001 to 10%, more preferably 0.005 to 5%, even more preferably 0.01 to 2%, further more preferably 0.05 to 1.5% and most preferably 0.1 to 1 % by total weight (w/w) of the composition.

12. The use of any one claims 1, and 3 to 11, or the pharmaceutical composition for use according to any one of claims 2 to 11, wherein the composition comprises a surfactant, wherein the surfactant is preferably present at 0.001 to 10%, more preferably 0.005 to 5%, even more preferably 0.01 to 2%, further more preferably 0.05 to 1.5% and most preferably 0.1 to 1% by total weight (w/w) of the composition.

13. The use of any one claims 1, and 3 to 12, or the pharmaceutical composition for use according to any one of claims 2 to 12, wherein the aqueous phase represents 15 to 99%, preferably 30 to 98%, more preferably 30 to 97%, and most preferably 70 to 95% by total weight (w/w) of the composition.

14. The use of any one claims 1, and 3 to 13, or the pharmaceutical composition for use according to any one of claims 2 to 13, wherein the composition comprises a preservative agent, wherein the preservative agent is preferably present at 0.001 to 10%, more preferably 0.005 to 5%, even more preferably 0.01 to 2%, further more preferably 0.05 to 1.5% and most preferably 0.1 to 1 % by total weight (w/w) of the composition.

15. The use of any one claims 1, and 3 to 14, or the pharmaceutical composition for use according to any one of claims 2 to 14, wherein the composition comprises a pH buffer, wherein the pH buffer is preferably present at 0,001 to 10%, more preferably 0.005 to 5%, even more preferably 0.0008 to 2%, further more preferably 0.01 to 1% and most preferably 0.1 to 1% by total weight (w/w) of the composition.

16. The use of any one claims 1, and 3 to 15, or the pharmaceutical composition for use according to any one of claims 2 to 15, wherein in the aqueous phase of the composition 25 to 50 ml/l, preferably 27,5 to 47.5 ml/l, more preferably 30 to 45 ml/l, even more preferably 32,5 to 43.5 ml/l and most preferably 39 to 40 ml/l nitrogen is solved.

17. A method for caring hair or skin comprising bringing into contact the cosmetic composition as defined in any one of claims 1, and 3 to 16 with skin or the scalp for a time which is sufficient to care the hair or skin.

18. The method of claim 17 or the use of any one of claims 1, and 3 to 16, wherein hair is cared by
(a) making up the keratinous fibers of hair, preferably human hair,
(b) inducing or stimulating the growth of the keratinous fibers of hair, preferably human hair,
(c) slowing down or preventing the loss of the keratinous fibers of hair, preferably human hair,
(d) increasing the density of the keratinous fibers of hair, preferably human hair; and/or
(e) increasing or maintaining the viability of keratinocytes, in particular of hair follicle kerationcytes.

19. The method of claim 17 or the use of any one of claims 1, and 3 to 16, wherein skin is cared by
(a) improving skin appearance, in particular scalp appearance, and/or
(b) reducing skin aging.

20. The pharmaceutical composition for use according to any one of claims 2 to 16, wherein the alopecia is androgenetic alopecia, alopecia areata, alopecia mucinosa, diffuse alopecia, alopecia actinica, alopecia mechanis, alopecia seborrhoica, alopecia parvimaculata, alopecia senilis, alopecia syphilitica, alopecia triangularis congenita Sabouraud, or alopecia congenita.

21. The pharmaceutical composition for use according to any one of claims 2 to 16, wherein the alopecia is caused by one or more of chronic inflammation, diabetes, dissecting cellulitis, a fungal infections (such as tinea capitis), hyperthyroidism and hypothyroidism, hypervitaminosis A, iron deficiency, lupus erythematosus, side effects from drugs (such as chemotherapy, anabolic steroids, and birth control pills), pseudopelade of brocq, radiation therapy, scalp infection (such as dermatophyte and tinea capitis), secondary syphilis, telogen effluvium, traction alopecia, trichotillomania and tufted folliculitis.

22. A method of manufacturing a composition as defined in any of claims 1 to 16, which comprises
(a) culturing stem cells in a medium,
(b) obtaining the supernatant of the stem cell culture of step (a),
(c) filling the supernatant of the stem cell culture obtained in (b) and the other ingredients of the composition into a pressure-resistant container, and
(d) applying gas to the composition during and/or after step (c).

23. The method of claim 21, wherein the gas is propan, butan, dimethylether or mixes, or NO or NO₂.

24. The method of claim 21 or 22, wherein the container is protected from light and consists of glass and/or polyethylene terephthalate (PET).
